Europäisches Patentamt

European Patent Office

Office européen des brevets

(11) Publication number: **0 350 701**
**A2**

# EUROPEAN PATENT APPLICATION

(21) Application number: 89111592.5

(22) Date of filing: 26.06.89

(51) Int. Cl.4: **A61K 9/16 , A61K 9/50**

(30) Priority: 12.07.88 IT 2132488

(43) Date of publication of application:
17.01.90 Bulletin 90/03

(84) Designated Contracting States:
AT BE CH DE ES FR GB GR IT LI LU NL SE

(71) Applicant: FARMA RESA S.r.l.
Via Como, 5
I-22063 Cantu' Como(IT)

(72) Inventor: **Conte, Ubaldo**
**Via Treviglio 6**
**I-21052 Busto Arsizio Varese(IT)**
Inventor: **Colombo, Paolo**
**Via Magenta 12**
**I-27100 Pavia(IT)**
Inventor: **La Manna, Aldo**
**Via Lanfranco 3**
**I-27100 Pavia(IT)**

(74) Representative: Gervasi, Gemma et al
NOTARBARTOLO & GERVASI Srl Viale
Bianca Maria 33
I-20122 Milan(IT)

(54) **Pharmaceutical cmpositions for oral administration having analgesic and anti-inflammatory activity, possessing excellent palatability and being free of irritating effects on mucous membranes.**

(57) Pharmaceutical compositions for oral administration in granular, or rapidly disgregating or effervescent tablets form to be dispersed in water at the time of the administration, comprising active substances having analgesic and anti-inflammatory activity which have irritating effects on the oro-pharingeal and gastric mucous membranes and unpleasent taste, and protective substances against said actions. The compositions of the invention have excellent palatability and are free of the irritating effect exerted by said active substances, without depressing the rapid achievement of therapeutically effective hematic levels.

EP 0 350 701 A2

## Pharmaceutical compositions for oral administration having analgesic and anti-inflammatory activity, possessing excellent palatability and being free of irritating effects on mucous membranes.

### Field of the invention

The present invention concerns pharmaceutical compositions for oral administration possessing analgesic and anti-inflammatory activity, to be dispersed in water at the time of administration.

### Prior art

The administration of drugs possessing analgesic and anti-inflammatory activity, such as non-steroid anti-inflammatory drugs (NSAD), is accompanied by irritant effects on the oro-pharingeal and gastric membranes, such effects being sometimes rather severe.

When such drugs are administered orally as solutions or acqueous dispersions an immediate burning feeling is perceived, which is particularly noticeable at the oro-pharingeal and gastric level.

Such effects are particularly undesirable when said drugs must be administered for long periods of time, such as for instance in the case of reumatic chronic syndromes, so that in certain clinical patterns, in the presence of gastro-duodenal ulcers and other gastro-irritant situations, the oral use of NSAD is drastically advised against.

A further problem which arises in the administration of NSAD is due to the fact that in the majority of cases a rapid therapeutic effect is desired and it is therefore necessary to rapidly reach a high plasmatic concentration.

Many solutions have been suggested and tested to the end of reducing the gastro-damaging effects of the above cited products.

In order to reach a rapid bio-availability, being the products mostly aryl-aliphatic, scarcely water-soluble, the use of their soluble salts such as the sodium-, lysine-, arginine-, aluminium-, etc. salts, has been suggested and effected; also in order to reduce the gastro-damaging effects, the use of pharmaceutical gastro-resistant formulas or the coating of the crystals and/or granules of the product with polymeric substances (such as ethylcellulose and similar products) in order to partially protect the gastric mucous membranes, was proposed and actuated. In most cases, however, such measures involve a slowing down of or a delay in the obtainment of therapeutically effective plasmatic levels.

Finally, a further inconvenience in the oral administration of most of the cited active products is their scarce palatability. Such drugs are therefore, also for this reason, not suitable for the preparation of pharmaceuticals for oral use and fast action, such as granules and/or effervescent tablets to be dispersed in water at the time of administration.

### Summary of the invention

We have now obtained pharmaceutical compositions for oral use, to be dispersed in water at the time of administration, in which the analgesic and anti-inflammatory active substance is in a form such as to obtain excellent palatability and to drastically reduce the irritating effect on the oro-pharingeal and gastric mucous membranes, while at the same time allowing to rapidly reach effective plasmatic levels.

The compositions according to the present invention are characterized by the fact that they contain the active substance in a finely divided form and coated by substances which allow to mask the taste of the active substance and to exert a protective action against its irritating effect on the oro-pharingeal and gastric mucous membranes.

Such compositions are prepared by a process characterized in that the active substance, in a finely divided state, is coated by substances able to mask the taste of the active substance and to exert a protective action against its irritating effect on the oro-pharingeal and gastric mucous membranes, and by the addition of substances known in the technique of preparing pharmaceutical compositions, obtaining compositions in granular form or in rapidly disgregating or effervescent tablets to be dispersed in water at the time of administration.

### Detailed description of the invention

2

The characteristics and advantages of the compositions according to the invention and the process for their preparation will be further explained in the following detailed description.

The compositions according to the invention comprise active substances having analgesic and anti-inflammatory activity, substances apt to mask the taste of the active substance and to protect the oro-pharingeal and gastric mucous membranes, and excipients normally employed in preparing pharmaceutical compositions.

As active substances there are employed non-steroid anti-inflammatory drugs, and in particular Ibuprofen, Naproxene, Ketroprofen, Suprofen, Indomethacin, Diclofenac, Diflunisal and their derivatives, as well as all active principles having similar characteristics.

Said products have a limited water solubility and/or present an unacceptable palatability while exerting a high irritating effect on the oro-pharingeal and gastric mucous membranes.

As protective substances of the mucous membranes there are employed aluminium hydroxides, sucralfate, other derivatives or aluminium and calcium salts, such as aluminium glycinate, activated attapulgite, aluminium hydroxide-magnesium carbonate gel, mucine, hydroxypropylmethylcellulose, acrylic and methacrylic acid copolymers and other natural and/or synthetic polymeric materials.

Said substances, beside having a protective action on mucous membranes, also serve to mask the taste of the active substance. In the compositions of the invention they coat the particles of the active substances and may be gastro-soluble and/or gastro dispersible, and in such case allow a rapid release of the active substance at the gastric level, or they can be gastro-resistant, but entero-soluble and/or entero-dispersible and in such case allow a rapid release of the active substance only at the enteric level.

Examples of gastro-soluble and/or gastro-dispersible substances are:

gelatin, arabic gum, acrylic and methacrylic acid derivatives (Eudragit E), carboxymethylcellulose and its derivatives, ethylcellulose, hydroxy-propyl-methyl-cellulose, polyoxyethylenglycols with different molecular weights, polyvinyl-alcohol and methylcellulose.

Examples of gastro-resistant but entero-soluble substances are:

cellulose acetophtalate, acrylic and methacrylic acid derivatives (Eudragit S, Eudragit L), polyvinyl aceto-phtalate, hydroxypropylmethylcellulose aceto-phtalate.

In the compositions according to the invention, the active substances are present in a finely divided state, the granules having in general dimensions lower than 100 microns and preferably comprised between 1 and 20 microns.

In any case, the active substance should have a granulometric distribution comprised within limits apt to obtain the attainment of the desired plasmatic concentration. In the case of Ibuprofen, for instance, the size of the granules will be comprised between 1 and 20 microns, preferably between 1 and 10 microns.

The weight ratio between active substance and protective substance of the mucous membranes is comprised between 10:1 and 1:10.

The compositions of the invention may also contain substances generally employed in the pharmaceutical technique, such as excipients, dispersing agents, sweetening agents, aromatic agents etc; they may contain for instance mannitol, sucrose, 80 polysorbate, lecithin, aspartame, ammonium glycyrrhizinate etc.

The process for coating, by the protective substance, the active substance particles and/or masking their taste may be realized in various ways, using the coating substances in a suitable form, e.g. aluminium hydroxide in colloidal form or as a humid gel and sucralfate in a micronized state or as a gel. The coating process may be carried out by simple mechanical mixing the various substances followed by screening between 500 and 1000 microns, oven drying in circulating air at 30-35 °C and a final screening between 200 and 700 microns to obtain a composition in granular form.

Another possible method consists of a coating process in an air fluidized bed at 30-40°C spraying in the fluidized bed a solution and/or a water suspension of the protective substance.

Still another method for coating the active substance with the protective substance consists in preparing a milky suspension of the composition as described and spray-drying it in air at 45-55°C.

In this way, a pulverized and/or granular product is obtained to which the necessary excipients for the desired pharmaceutical composition are added.

Said excipients may be:

sweetening agents (natural and/or synthetic), aromatizing agents (natural and/or synthetic), lubricants and gliding agents and other adjuvant substances according to known techniques.

The compositions according to the invention are prepared in granular form to be distributed in sachets, or as rapidly disgregating or effervescent tablets for oral use dispersible in water at the time of administration.

They easily disperse in water forming milky suspension having pleasant odour and taste.

Said compositions have the following characteristics:

3

they have pleasant organoleptic properties which favor their use in the pediatric and geriatric employment;
- they do not have any irritating effect although comprising an active substance having per se an irritating effect on the oro-pharingeal and gastric mucous membranes;
- they allow to reach high hematic levels in a very reduced time.

By the way of non-limiting illustration we report herein below examples on the preparation and use of the compositions according to the invention.

Example 1

A granular composition is prepared, in which the various components are contained in the following percentages by wt.

| Ibuprofen | 5.65% |
|---|---|
| Al-hydroxide, colloidal | 5.65% |
| Mannitol | 39.50% |
| Sucrose | 39.55% |
| Polysorbate 80 | 0.90% |
| Ammonium glycyrrhizinate (Glycamil) | 2.80% |
| Aromatizing agent | 5.90% |

The Ibuprofene granules had a dimension of less than 10 microns: it was admixed with the polysorbate in a mortar, then colloidal aluminium hydroxide and mannitol were added, after screening on a 710 microns screen, the product was dried in a oven under air circulation at 30-35°C. After a new screening on a 600 microns screen the remaining components were added and the compound was carefully mixed.

Sachets were prepared with the obtained granular product, each containing an amount of granules corresponding to 200 mg Ibuprofen.

Tests "in vitro"

The granular product disperses easily in water forming a milky suspension having pleasant odour.
No sediment is noted to any appreciable extent: any sediment formed may in any case be easily re-dispersed again.

Tests "in vivo"

a- Palatability

The product dispersed in water had excellent palatabiliy characteristics.
The taste of the active principle is totally masked. No irritating effect on the oro-pharingeal level, typical of the Ibuprofen is noted.

b- Plasmatic levels

An amount of product corresponding to 200 mg Ibuprofen dispersed in 100 ml water was administered to healthy volunteers, and the plasmatic levels at various times were determined.

| Time (minutes) | Plasmatic concentration (mcg/ml) |
|---|---|
| 15 | 9.291 |
| 30 | 16.217 |
| 60 | 18.502 |
| 120 | 18.502 |
| 240 | 9.956 |
| 360 | 4.050 |

The obtained results show that the administered composition was able to establish high plasmatic levels within 15' after the administration.

For comparison purposes to the same subjects were administered commercial Ibuprofen 200 mg tablets. The resulting plasmatic levels, similarly determined, are reported below.

| Time (minutes) | Plasmatic Concentration (mcg/ml) |
|---|---|
| 15 | - |
| 30 | 2.48 |
| 60 | 3.30 |
| 120 | 24.01 |
| 240 | 14.17 |
| 360 | 4.94 |

The obtained results confirm that the granular composition according to the present invention is apt to produce effective plasmatic levels within 15-30', while the commercial tablets reach the same levels in a decidedly longer time.

Example 2

A granular composition is prepared in which the various components are contained in the following percentages (by wt.)

| | |
|---|---|
| Ibuprofen | 5.33% |
| Al hydroxide humid gel, (Armour-Reheis) | 10.66% |
| Mannitol | 37.33% |
| Sucrose | 37.33% |
| Polysorbate 80 | 0.53% |
| Lecithin | 0.26% |
| Ammonium glycyrrhizinate (Glycamil) | 1.86% |
| Citric acid | 3.26% |
| Aromatizing substance | 3.44% |

Ibuprofen is mixed in a mortar with the polysorbate and lecithin. The aluminium hydroxide humid gel and the mannitol are added.

After screening at 710 microns, the product is dried in a oven at 30-35° C under air circulation, the other components are added and the compound is screened at 300 microns.

From the granular product sachets are prepared each containing an amount of granular product corresponding to 200 or 400 mg

Ibuprofen.

Example 3

A granualr composition is prepared with the following substances (wt.%)

| Ibuprofen | 4.0% |
| Sucralfate | 4.0% |
| Mannitol | 40.0% |
| Sucrose | 40.0% |
| Dioctyl sulfosuccinate Na | 0.2% |
| Anti-foam (Wacker VP 1144) | 0.2% |
| Aspartame | 3.0% |
| Citric acid | 3.0% |
| Aromatizing excipients | 5.6% |

Ibuprofen is introduced in a mixer and moistened with a 10% solution of sodium dioctylsulfosuccinate, a 20% suspension Sucralfate gel is added and the volume is brought to 100 ml water; after adding the anti-foaming agent, the milky suspension obtained is dried in a Buchi spray-dryer in air at 50° C. A dry, easily flowing homogeneous product is obtained. The remaining components are then added and sachets of the granular product are prepared each containing an amount of granular product corresponding to 200 mg or 400 mg Ibuprofen.

Example 4

A product in the form of effervescent tablets is prepared containing the following components:

| Ibuprofen | 200 mg |
| Sodium Dioctylsulfosuccinate | 20 mg |
| Lecithin | 5 mg |
| Aluminium hydroxide | 50 mg |
| Anti-foam (Wacker VP1144) | 5 mg |
| Anhydrous tartaric acid | 700 mg |
| Sodium bicarbonate | 950 mg |
| Polyoxyethylenglycol | 20 mg |
| Aspartame | 50 mg |
| Aromatizing excipients | 155 mg |

Ibuprofen, sodium dioctylsulfosuccinate, lecithin and the anti-foam agent are mixed together in a mixing apparatus. Aluminium hydroxide gel is added. After further mixing, the homogeneous mix is screened through a 710 microns screen; the granular product is dried in air in an oven at 30-35° C.

To the dried granules the remaining components are added and the product is mixed further for 20 minutes until homogeneous. The granular product is pressed according to known technique using a 20 mm diameter punch, operating in a room with a relative humidity below 40%.

Tablets are thus obtained each containing an amount of product corresponding to 200 mg Ibuprofen.

Example 5

A granular composition is prepared containing as active substance the S-enantiomer of Ibuprofen and in which the components are present in the following amounts: (wt %)

6

| S-Ibuprofen | 5.33% |
|---|---|
| Al-hydroxide humid gel | 10.66% |
| Mannitol | 37.33% |
| Sucrose | 37.33% |
| Polysorbate 80 | 0.53% |
| Lecithin | 0.26% |
| Ammonium glycyrrhizinate (Glycamil) | 1.86% |
| Citric acid | 3.26% |
| Aromatizing excipients | 3.44% |

The pure S-Ibuprofen enantiomer is admixed in a mortar with the polysorbate and lecithin.

Aluminium hydroxide humid gel and mannitol are added, the mixture is screened on a 710 microns screen, the product is dried in an oven, in air at 30-35°C, the remaining components are added and the compound is screened on a 300 microns screen. The compound is mixed and sachets of the granular product are prepared each containing an amount of granular product corresponding to 100, 200 or 400 mg S-Ibuprofen.

Example 6

A granular composition is prepared containing (wt %)

| Naproxen | 5.0% |
|---|---|
| Sucralfate | 5.0% |
| Mannitol | 40.0% |
| Sucrose | 40.0% |
| Na-Dioctylsulfosuccinate | 0.2% |
| Anti-foam (Wacker VP 1144) | 0.2% |
| Aspartame | 3.0% |
| Citric acid | 3.0% |
| Aromatizing excipients | 5.6% |

The finely milled Naproxen is put into a mixer and mixed with a sodium dioctylsulfosuccinate 10% water solution, a sucralfate gel 20% suspension is added and water is added up to about 100 ml. After adding the anti-foam agent, the milky suspension obtained is dried in a Buchi spray-dryer in air at 50°C. A dry, easily flowing, homogeneous product is obtained.

The remaining components are added and sachets of the granular product are prepared, each containing an amount of granular product corresponding to 200 and 400 mg Naproxen.

Example 7

Effervescent tablets are prepared with a composition consisting of:

| Dichlofenac | 100 mg |
| Sodium laurylsulphate | 20 mg |
| Lecithin | 5 mg |
| Aluminium hydroxide gel | 50 mg |
| Anti-foam (Wacker VP1144) | 5 mg |
| Anhydrous tartaric acid | 800 mg |
| Sodium bicarbonate | 950 mg |
| Polyoxyethylenglycol 6000 | 20 mg |
| Aspartame | 50 mg |
| Aromatizing excipients | 155 mg |

Dichlofenac is admixed with the sodium laurylsulphate, lecithin and the anti-foam agent in a mixing apparatus.

Aluminium hydroxide gel is added.

After mixing, the homogeneous product is screened on a 710 microns screen.

The granular products is dried in an oven in air at 30-35° C.

The remaining components are added to the dry granules and the mixture is mixed for further 20 minutes until homogeneous.

The granular product is pressed according to the known technique using a 20 mm diameter punch and operating in a room with a relative humidity below 40%.

Tablets are obtained, each containing an amount of product corresponding to 100 mg Dichlofenac.

Said tablets when placed in a small amount of water give a rapid effervescence and a milky dispersion is obtained, which does not irritate the oro-pharingeal mucous membranes and has an excellent palatability.


## Claims

1. Pharmaceutical compositions for oral use having analgesic and anti-inflammatory activity to be dispersed in water at the time of administration, characterized by the fact that they comprise the active substance in a finely divided form and coated by substances apt to mask the taste of the active substance and to exert a protective action against the irritating effect of the active substance on the oro-pharingeal and gastric mucous membranes.

2. Compositions according to claim 1, characterized in that said active substance is an anti-inflammatory non-steroid drug.

3. Compositions according to claim 2, characterized in that said active substance is selected from the group consisting of: Ibuprofen, Naproxen, Ketoprofen, Suprofen, Indomethacin, Dichlofenac, Diflunisal or a derivative of anyone of these substances.

4. Compositions according to claim 1, characterized in that the dimensions of the granules of said active substance are below 100 microns and preferably comprised between 1 and 20 microns.

5. Compositions according to claim 1, characterized in that said substance having a protective action on mucous membranes is selected from the group consisting of aluminium hydroxide, sucralfate, other derivatives or aluminium or calcium salts, such as aluminium glycinate, activated attapulgite, aluminium hydroxide-magnesium carbonate gel, mucin, hydroxypropylmethylcellulose, an acrylic acid and methacrylic acid copolymer.

6. Compositions according to claim 1, characterized in that the weight ratio between said active substance and said protective substance is between 10:1 and 1:10.

7. Compositions according to claim 1, characterized in that in addition to said active substance and said protective substance, they contain substances commonly used in the preparation of pharmaceutical compositions, such as excipients, dispersing agents, sweetening and aromatizing agents.

8. Compositions according to claim 7, comprising mannitol, sucrose, polysorbate 80, aspartame, lecithin, ammonium glycyrrhizinate.

9. Compositions according to claim 1, characterized by being prepared in granular form to be packed in sachets.

10. Compositions according to claim 1, characterized by being prepared as rapidly disgregating or effervescent tablets to be dispersed in water for oral use at the time of administration.

11. A process for the preparation of pharmaceutical compositions for oral use, having analgesic and anti-inflammatory activity, characterized in that the active substance in finely divided form with a granulometry below 100 microns is coated by substances apt to mask the taste of the active substance and to exert a protective action against the irritating effect, produced by the active substance on the oro-pharingeal and gastric mucous membranes, that substances known in the technique of preparing pharmaceutical compositions are added, that the obtained compositions are made into granular form or as rapidly disgregating or effervescent tablets to be dispersed in water for oral use at the time of administration.

12. A process according to claim 11, characterized in that said substances employed in the coating treatment are selected from the group consisting of: alluminium hydroxide in colloidal or humid gel form, sucralfate in micronized or in gel form, mucine, hydroxypropylcellulose, acrylic and methacrylic acid copolymers.

13. A process according to claim 11, characterized in that said coating treatment is carried out by mechanical mixing the components, followed by screening between 500 and 1000 microns, drying at 30-35° C and final screening between 200 and 700 microns.

14. A process according to claim 11, characterized in that said coating treatment is carried out in a air-fluidized bed at 30-40° C, feeding to the fluidized bed a water suspension of the coating substance.

15. A process according to claim 11, characterized in that said coating process is carried out by spray-drying in air at 45-55° C a milky suspension of the composition.